# EUROPEAN PATENT SPECIFICATION

(11) **EP 2 932 239 B1**
(45) Date of publication and mention of the grant of the patent: **18.03.2020**
(21) Application number: 12806014.2
(22) Date of filing: 13.12.2012
(51) Int. Cl.: G01N 21/35, G01N 33/28, G01N 33/30, G01N 21/88

(54) **SENSOR ARRAY FOR OIL SATURATION MEASUREMENT**
SENSORANORDNUNG ZUR MESSUNG DER ÖLSÄTTIGUNG
RÉSEAU DE CAPTEURS POUR MESURE DE SATURATION D'HUILE

(43) Date of publication of application: 21.10.2015
(73) Proprietor: Aktiebolaget SKF, 415 50 Göteborg (SE)
(72) Inventor: TATAR, Florin, NL-2622 GE Delft (NL); HERDIER, Romain, B-1180 Uccle (BE)
(74) Representative: Kuhstrebe, Jochen
(86) International application number: PCT/EP2012/075395
(87) International publication number: WO 2014/090309

(56) References cited:
- WO-A2-2011/073789
- DE-A1- 3 712 879
- DE-A1- 10 053 069
- US-A1- 2007 114 372
- US-A1- 2010 208 261

## Description

### BACKGROUND OF THE INVENTION

### 1. Field of the Invention

The present invention relates to oil condition monitoring and in particular, to a sensor for detecting the presence of water in oil or like substances. The invention also relates to a method of monitoring the presence of water in oil and in particular to a method which allows easy auto-calibration of the sensor in-situ and low-cost implementation.

### 2. Description of the Related Art

Optical sensors have been used for oil condition monitoring for determining the presence of debris or otherwise monitor deterioration of a lubricant. Such devices may operate by shining light through a small gap and analysing the transmitted light with a suitable optical sensor. Alternative sensors may make use of scattering of light and may operate over different frequencies including outside of the visible range. Oil condition monitoring may be significant in providing feedback in advance of likely failure of a lubricant system. Action may be taken to perform maintenance or otherwise renew the lubricant.

Water in oil is of considerable concern to many mechanical systems. Minimal amounts of water may be absorbed by the oil during use, either from the atmosphere or by direct ingress of water into the system. As long as this water is in the absorbed state and the oil is unsaturated, the concern is minimal. Nevertheless, as the concentration of water approaches the saturation level, emulsified and free water may occur, which can be highly detrimental, especially if exposure is prolonged. In bearings, the incompressibility of water relative to the oil can result in disruption of the oil film leading to excessive wear. Just one percent water in oil can reduce the life expectancy of a bearing by as much as 90 percent. For ball or rolling element bearings, the localized pressure generated can cause spontaneous vaporization of the water, leading to erosive wear such as micropitting. The saturation level of water in oil may vary widely according to temperature and the type of oil and can range from 10 ppm to even 10000 ppm. Existing sensors capable of measuring the presence of water (free and dissolved) include capacitive sensors and Karl Fischer titration sensors. Both of these methods require considerable time for the sensor to reach equilibrium and are not ideal for rapidly changing conditions. Spectral analysis using Fourier Transform Infrared Spectroscopy (FTIR) has been used but is a relatively complex and costly procedure requiring calibration of the sensor relative to the spectrum produced with fresh oil. Spectral analysers, such as the one known from US 2007/114372 A1, are also relatively costly, bulky and sensitive devices for installation in many environments where mechanical systems are located.

It would thus be desirable to provide for a low-cost and simple sensor arrangement that could reliably identify the presence of water in oil in real time.

### BRIEF SUMMARY OF THE INVENTION

According to the invention there is provided a system for detecting the presence of water in oil, comprising: an array of infra-red emitters for emitting light at a first plurality of wavelengths between 1100 nm and 1500 nm; an array of infra-red receivers, each arranged to detect light from a respective emitter at the respective wavelength and produce a light signal representative of the light detected, the receivers being spaced from the emitters by a gap through which oil in use may pass; and a processor arranged to analyse and compare the respective light signals to determine an amount of water present in the oil. According to the invention, it has been shown that the amount of water in the oil can strongly affect the absorption of light in the near infra-red spectrum. By observing variations in absorption, the presence of water can be identified. Determination of the amount of water may be made on the basis of previously calibrated values. It has further been shown that by operating at a number of wavelengths across the infra-red region, relatively reliable determination can be achieved. Still according to the invention, the processor is arranged to determine when a fluctuation of one or more of the light signals exceeds a preset value.

In a preferred embodiment of the invention, the gap separating the receivers from the emitters is less than 2 mm, preferably less than 1 mm and most preferably around 0.5 mm. It has been found that increase light sensitivity may be achieved with a smaller gap. Nevertheless, a gap of around 0.5 mm is considered optimal, as it allows more than sufficient light transmission for most oils and is not so narrow that clogging with debris could occur. It will be understood that for relatively more viscous oils a larger gap might be considered than for lighter oils.

A matched emitter and receiver will henceforth be referred to as a sensor. The skilled person will be aware that various forms of sensor may be used. Most preferably, the emitters are light emitting diodes and the receivers are photodiodes, operating at the corresponding frequency. Such devices are relatively cheap and reliable allowing oil condition monitoring to be implemented in situations not otherwise contemplated.

In a particularly advantageous embodiment, the emitters and the receivers are remote from the gap and not in direct contact with the oil. In such an embodiment, the emitters may be coupled into an input of an emitter optical fibre and the receivers may be coupled with an output of a receiver optical fibre, with the gap being provided between an output of the emitter optical fibre and an input of the receiver optical fibre. According to this embodiment, it has been found significantly easier to couple the light from the emitter optical fibre to the receiver optical fibre and scattering of light is reduced. Each sensor pair may be provided with a dedicated optical fibre connection. Alternatively, the emitters may be coupled into the input of the emitter optical fibre via an emitter optical switch and the receivers may be coupled with the output of the receiver optical fibre via a receiver optical switch. In this case, the emitter optical switch and the receiver optical switch should be synchronised to each other to ensure that the respective receiver detects light from the matched emitter. An additional advantage of such a configuration is that the optical fibres may be integrated within a mechanical system where the gap is located and the emitters and receivers may be located remotely at an accessible location allowing exchange and/or addition of sensors without otherwise disturbing the system.

According to the invention, it has been observed that a significant change in signal characteristic is to be observed at the point at which free water appears in the oil. Below the saturation level, the light signal as received by a receiver is relatively stable and only steadily decreases in intensity with increasing absorbed water content. As the amount of water approaches saturation, the light signal becomes highly unstable and may appear noisy. Without wishing to be bound by theory, it is believed that bubbles of free water are formed within the oil in a manner similar to cavitation or boiling of a liquid. As these bubbles pass through the gap, they disturb the signal, effectively leading to greater absorption of the light and a lower light signal. A significant advantage of the above effect is that the system can be easily calibrated in-situ to the saturation level, without requiring knowledge of either the oil or receiver characteristics. Additionally, the system can provide real-time results with negligible delay in identifying the presence of free water in the oil

According to a further aspect of the invention, the processor is arranged to compare the respective light signals and determine the amount of water based on the light signals of a sub-group of the sensors. Because the absorption properties of different oils may vary, not all of the sensors may register the presence of water to the same extent. By comparing a number of sensors, a more accurate determination may be made. In one embodiment, identification of water may be made when at least two, or preferably at least three of the sensors provide corresponding outputs. The signals may be combined and compared in any appropriate manner including by the use of hardware such as bridge circuits and using software algorithms. The processor may be any appropriate processing device such as a computer or dedicated microprocessor. In addition to other control tasks, the processor is preferably arranged to determine when the fluctuation of the light signal exceeds the preset value. In particular the processor may carry out signal analysis, sampling and filtering as required.

It has been found that detection is particularly effective for infra red light in the range from 850 nm to 1750 nm. Near infra red light shows good absorption properties for water while being only slightly affected by other contaminants in the oil. According to the present invention, the first plurality of wavelengths lie between 1100 nm and 1500 nm. In this region, excellent absorption properties of water in oil have been observed. Most specifically the sensors may be operate around 1250 nm and 1400 nm. These wavelengths correspond to characteristic peaks in absorption for many oils. Nevertheless, the exact wavelength of such peaks varies from oil to oil and for this reason it is desirable to have a plurality of sensors operating close to these values. As an example, four sensors may be provided operating at wavelengths of around 1240 nm, 1260 nm, 1390 nm and 1410 nm respectively. The skilled person will understand that this is merely exemplary and the best wavelengths will be determined by trial and error or by pre-calibration. Additionally, the choice of wavelength will depend on the bandwidth sensitivity of the sensors. when at least two, or at least three of the sensors register a corresponding decrease of the light signal due to absorption or detect the presence of fluctuations.

The skilled person will understand that the sensor of the present invention may be implemented in a number of different situations where water in oil is to be recorded. Preferably, the optical sensor is located in an oil supply line to a mechanical system. The mechanical system may be a motor, a gear, a bearing, a journal, a cam or a complex system comprising one or more of the above

The invention also relates to a method for detecting the presence of water in oil using the system as described above, comprising passing light through the oil from the emitters to the receivers; detecting at the receivers, the light transmitted from the emitters to produce light signals representative of the light detected; and analysing and comparing the respective light signals to determine an amount of water present in the oil. Determination of the amount of water can be realised in many ways as will be evident to the skilled person. This may be determined qualitatively by manually reviewing a data stream record of the light signals. Alternatively and preferably, the method may be carried out by the processor using an appropriate algorithm.

In one embodiment of the invention, the method comprises analysing the light signals to determine an amount of fluctuation of the signals; monitoring the amount of fluctuation of the light signals to identify a step change representing saturation of the oil and generating a saturation signal indicative of saturation when at least one of the light signals indicates fluctuation of more than a preset value.

The amount of fluctuation may be determined by measuring a maximum peak to peak variation of the light signal within a sampling period. The sampling period may be chosen depending on various factors, including the sampling rate at which measurements of the light signal are taken and also based on physical factors such as the flow rate of the oil being monitored. It will be understood that the sampling period will include at least two samples, preferably at least four samples and more preferably at least 10 samples. The sampling period may be between 0.5 seconds and 10 seconds, preferably between 1 second and 3 seconds A sample rate of between 1 Hz and 10 Hz may be used, preferably around 2Hz, again depending on the flow rate. In general, for a higher flow rate of the oil, a higher sample rate may be required for the same sensitivity.

According to one method of analysis, saturation may be identified when a ratio of the light signal fluctuation in a second sampling period to the light signal fluctuation in a first sampling period exceeds a predetermined value. The predetermined value may be determined according to the nature of the oil and other physical factors. In general, with the onset of free water the signal fluctuation may increase ten-fold or more and a predetermined value of 5 may be sufficient to provide reliable indication while avoiding false alarms. In other circumstances, a predetermined value of 2 may provide more sensitive response and predetermined values of below 2 may be applicable, in particular where signal smoothing has previously been applied.

According to a further aspect of the invention, the method may further comprise determining the time that the oil remains above its saturation level. Once free water is detected in the oil, a timer may register the time elapsed until the danger of free water has receded. This point may be determined by evaluating a number of successive sampling periods and determining that water is absent once absence of saturation signal has been determined for all of these periods. Alternatively, once an absolute value of the light signal corresponding to the saturation level has been determined, the absence of free water may be indicated once the absolute value of one or more of the light signals returns to a value distant from the saturation level.

According to an alternative method, the exposure of the system to free water may be determined by integrating the saturation signal with respect to time. Based on the flow rate of the oil through the sensor, integration of the signal may allow an approximate determination of the total amount of free water in the system. This may be used to provide further alarms and initiate appropriate actions in the event that a given exposure is exceeded.

According to the invention, it has been found that the method is particularly effective for infra red light in the range from 850 nm to 1750 nm. Near infra red light shows good absorption properties for water while being only slightly affected by other contaminants in the oil. The light may be analysed over a broad spectrum e.g. using a spectroanalyser. Most preferably, the light is analysed in regions of the spectrum where water absorption peaks are present. For most oils, such absorption peaks may be identified at around 1250 nm and around 1400 nm and analysis in one or both of these specific regions is preferred.

A significant advantage of the present invention is that the sensors need not be pre-calibrated and may be calibrated in-situ based on recognition of the saturation level. In the event that greater accuracy is required in the region of absorbed water, the method may comprise calibrating the sensors for a sample of oil having a water content below the saturation level and subsequently determining a linear relation between the light signal and the water content when saturation of the oil is detected. Such a simple calibration may be achieved in a laboratory by calibrating the sensors against a Karl Fischer titration result. Alternatively, the sensors may be calibrated in the field by taking an oil sample for off-line analysis. Once calibrated, the sensors may be accurately used to also identify emulsified water in oil before the advent of free water.

The processor may be any appropriate processing device such as a computer or dedicated microprocessor. In addition to other control tasks, the processor is preferably arranged to determine when the fluctuation of the light signal exceeds the preset value. In particular the processor may carry out signal analysis, sampling and filtering as described above.

### BRIEF DESCRIPTION OF THE DRAWINGS

The features and advantages of the invention will be appreciated upon reference to the following drawings of a number of exemplary embodiments, in which:
Figure 1 shows a schematic view of an experimental setup for determining the absorption spectrum of an oil sample;
Figure 2 shows a graph of light intensity signal detected by the system of Figure 1 over the range of wavelengths from 850nm to 1700 nm;
Figure 3 shows light intensity measurements for increasing water content measured in the system of Figure 1 for the wavelength of 1108 nm;
Figure 4 shows relative humidity measurements for the system of Figure 1 in parallel with commercial sensors; and
Figure 5 shows a system according to the present invention.

### DESCRIPTION OF ILLUSTRATIVE EMBODIMENTS

### Example 1

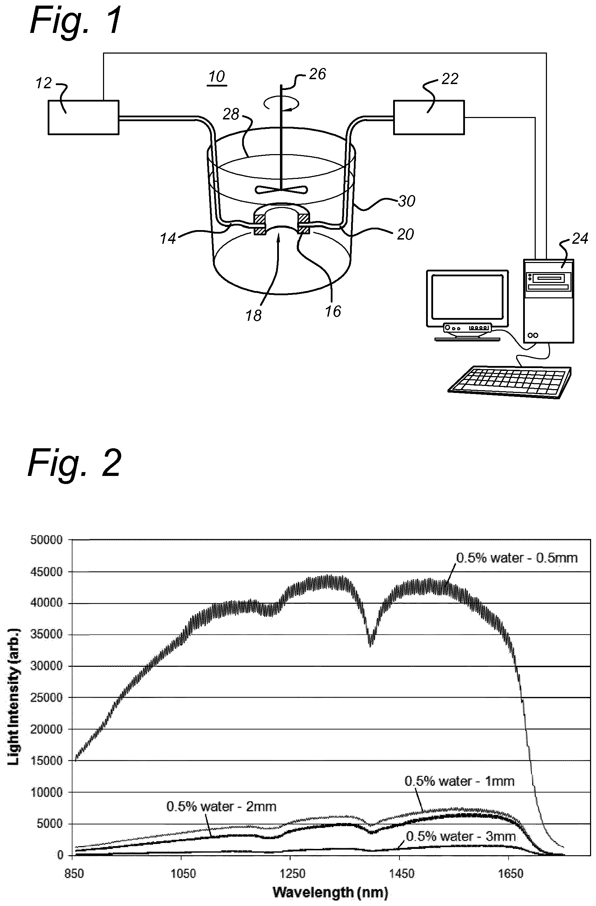

Figure 1 shows a schematic view of an experimental set up of a system 10 for determining the absorption spectrum of an oil sample. The system comprises a deuterium/halogen lamp source 12 coupled to an optical fibre emitter 14. The optical fibre emitter 14 is coupled to a detection cell 16 comprising a gap 18. Facing the optical fibre emitter 14 across the gap 18 is an optical fibre receiver 20 coupled to a spectroanalyser 22. The spectroanalyser 22 is in turn connected to a personal computer 24. Both the optical fibre emitter 14 and optical fibre receiver 20 comprised optical fibres of 400 mm length. A stirrer 26 is provided to circulate the oil 28 contained in beaker 30.

In an initial test, the effects on the system 10 with regards to the width of the gap 18 within the detection cell 16 were investigated. For this a number of different detection cells 16 were constructed having gap widths of 3mm, 2mm, 1mm and 0.5mm respectively. For this example, the system 10 of Figure 1 was applied to a bath of mineral oil (Total Cirkan C100™), placed in a controlled humidity/temperature chamber. Figure 2 illustrates the resulting light intensity signal detected by the spectroanalyser 22 over the range of wavelengths from 850nm to 1700 nm, the light intensity being given on an arbitrary scale from 0 to 50 000. As can be seen, the light intensity measurements for the 0.5 mm gap are considerably higher than for the larger gaps and also offer a significantly greater signal to noise ratio for subsequent analysis. Also visible are dips in the light intensity measurements at 1240nm and 1400nm respectively. The dip at 1400 nm is particularly pronounced for this oil and represents strong adsorption due to the presence of water.

### Example 2

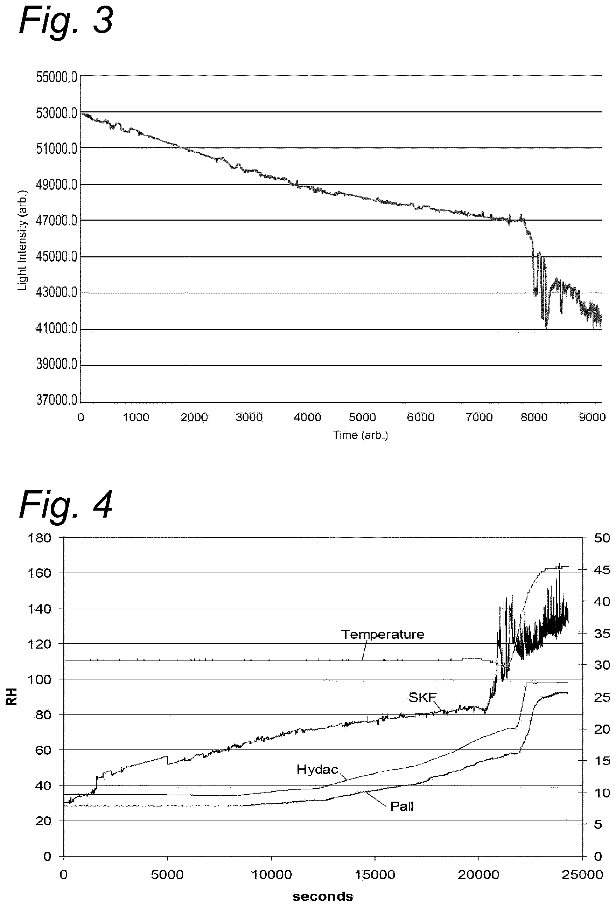

In a subsequent test, the same system 10 was used as in Example 1, whereby the 0.5 mm gap detection cell 16 was employed. The full spectrum was recorded over a number of hours, during which the concentration of the water was steadily increased until the saturation level was reached (100% saturated). After this the water concentration was further increased to about 0.4% water. The light intensity measurements for the full experimental test are shown in Figure 3 for the wavelength of 1108 nm. The result shows a strong and stable response to the ever increasing levels of water content. As the sample reaches saturation, the sensor records significant fluctuation in the signal. The sampling rate was 2 Hz. The skilled person will recognize that digital filtering such as a moving point average could be used to further smooth the signal.

### Example 3

The measurements as performed in Example 2 were repeated in a controlled climate chamber. By adjusting the humidity steadily, it could be assumed that the oil would be in equilibrium with the air within the chamber and have the same relative humidity. The measurements were performed in parallel with commercial (Hydac and Pal) capacitance sensors which absorb or desorb moisture, to achieve equilibrium with the surrounding fluid. The results are displayed in Figure 4 in which the relative humidity value is based on the reading for the controlled climate within the chamber. After 3 hours of recording, the Hydac and Pal sensors did not show significant variation of the saturation measured values (30% and respectively 35%), while the detection cell 16 indicated 70% saturation. At this point, visual observation of the oil showed that it had changed from very transparent to non-transparent. The atmospheric humidity of the chamber was 100% humidity. Following further measurements, when the oil reached a milky observed colour indicating the emulsified state, the detection cell 10 registered absolute humidity of 100%. At this point, the Pal and Hydac sensors registered within the 50-70% range respectively. The results as plotted in Figure 4 appear to show a delay of 1 to 3 hours for these sensors with respect to the actual values. It is also noted that beyond saturation in the free water region, the detection cell 16 continues to show variation and allows calibration and measurement in this region. The Pal and Hydac sensors reach a plateau at saturation and do not register the free water content.

The above tests were repeated for polyglycol with similar results. Based on the above it appears that the sensor portrays a significant level of sensitivity (20ppm), a wide range of detection (20ppm - 10000ppm) and fast response and recovery times.

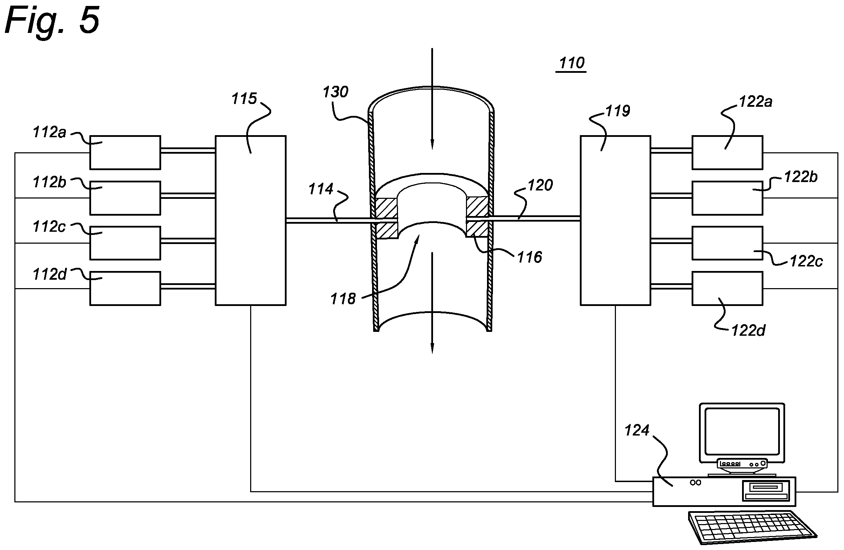

Figure 5 shows a schematic view of a system 110 according to the present invention, comprising an array of emitters 112A-D coupled to an emitter optical fibre 114 via an emitter optical switch 115. The emitter optical fibre 114 is coupled to a detection cell 116 comprising a gap 118. Facing the emitter optical fibre 114 across the gap 118 is a receiver optical fibre 120, which is coupled via a receiver optical switch 119 to an array of receivers 122A-D. The receivers 122A-D are in turn connected to a personal computer 124. Both the emitter optical fibre 114 and receiver optical fibre 120 comprised optical fibres of 400 mm length. The detection cell 116 is integrated into an oil supply line 130.

In a prophetic example utilising the system 110 of Figure 5, the sensor pairs 112A-D, 122A-D operate at 1230 nm, 1260 nm, 1390nm and 1410 nm. The system is set to sample at a rate of 2Hz with the block of optical switches 115, 119 operating at low power with a switch time of around 20ms. The switches 115, 119 are MEMS, although prism or rotating optical switches may also be used. The synchronization of the sensor pairs 112A-D, 122A-D is performed by the personal computer 124 which controls the optical switches. The personal computer 124 is programmed to measure a maximum peak to peak variation of each received light signal within a sampling period of 2 seconds. A saturation signal is given for a sensor when a ratio of the light signal fluctuation in a second sampling period to the light signal fluctuation in a first sampling period exceeds a predetermined value of 2. When at least three of the four sensors indicate saturation, a saturation alarm is given indicative of the presence of free water in the oil. The absolute values of the light signal for the respective first sampling period (prior to the onset of saturation) is stored in a memory of the computer 124 as a calibrated value for 100% saturation.

Thus, the invention has been described by reference to the embodiment discussed above. It will be recognized that this embodiment is susceptible to various modifications and alternative forms well known to those of skill in the art without departing from the scope of the invention. In particular, it will be understood that many different algorithms and signal analysis procedures may be carried out on to determine the oil condition based on the sensor outputs. Furthermore, the personal computer may be replaced by a dedicated microprocessor which may be located in-situ or remotely. Accordingly, although specific embodiments have been described, these are examples only and are not limiting upon the scope of the invention.

## Claims

1. A system for detecting the presence of water in oil, comprising:
an array of infra-red emitters (112A, 112B, 112C, 122D) for emitting light at a first plurality of wavelengths between 1100 nm and 1500 nm;
an array of infra-red receivers (122A, 122B, 122C, 122D), each arranged to detect light from a respective emitter at the respective wavelength and produce a light signal representative of the light detected, the receivers being spaced from the emitters by a gap (118) through which oil in use may pass; and
a processor (124) arranged to analyse and compare the respective light signals to determine an amount of water present in the oil, wherein the processor (124) is arranged to determine when a fluctuation of one or more of the light signals exceeds a preset value.

2. The system of claim 1, wherein the gap (118) separating the receivers from the emitters is less than 2 mm, preferably less than 1 mm and most preferably around 0.5 mm.

3. The system of claim 1 or claim 2, wherein the emitters (112A, 112B, 112C, 112D) are light emitting diodes and the receivers are photodiodes (122A, 122B, 122C, 122D), operating at the corresponding frequency.

4. The system according to any preceding claim, wherein the emitters are coupled into an input of an emitter optical fibre (114) via an emitter optical switch (115), the receivers are coupled with an output of a receiver optical fibre (120) via a receiver optical switch (119), the gap (118) is provided between an output of the emitter optical fibre and an input of the receiver optical fibre and the emitter optical switch and the receiver optical switch are synchronised to each other.

5. The system according to any preceding claim, wherein the processor is arranged to compare the respective light signals and determine the amount of water based on the light signals of a sub-group of the sensors.

6. The system according to any preceding claim, wherein the first plurality of wavelengths are around 1250 nm and 1400 nm.

7. The system according to any preceding claim, wherein the arrays comprise at least four emitters (112A, 112B, 112C, 112D) and at least four receivers (122A, 122B, 122C, 122D).

8. The system according to any preceding claim, located in an oil supply line to a mechanical system.

9. A method for detecting the presence of water in oil using the system according to any preceding claim, comprising passing light through the oil from the emitters (112A, 112B, 112C, 112D) to the receivers (122A, 122B, 122C, 122D) detecting at the receivers, the light transmitted from the emitters to produce light signals representative of the light detected, analysing and comparing the respective light signals to determine an amount of water present in the oil, analysing the light signals to determine an amount of fluctuation of the signals, monitoring the amount of fluctuation of the light signals to identify a step change representing saturation of the oil and generating a saturation signal indicative of saturation when at least one of the light signals indicates fluctuation of more than a preset value.

10. The method of claim 9, wherein determining an amount of fluctuation comprises measuring a peak to peak variation of the light signal within a sampling period.

11. The method according to claim 10, wherein the sampling period is between 0.5 seconds and 10 seconds, preferably between 1 second and 3 seconds.

12. The method according to claim 10 or claim 11, wherein saturation is identified when a ratio of the light signal fluctuation in a second sampling period to the light signal fluctuation in a first sampling period exceeds a predetermined value.

13. The method according to any of claims 9 to 12, further comprising determining the time that the oil remains above its saturation level.

## Patentansprüche

1. System zum Detektieren des Vorhandenseins von Wasser in Öl, umfassend:
eine Anordnung von Infrarotstrahlern (112A, 112B, 112C, 122D) zum Abstrahlen von Licht bei einer ersten Mehrzahl von Wellenlängen zwischen 1100 nm und 1500 nm;
eine Anordnung von Infrarotempfängern (122A, 122B, 122C, 122D), die jeweils angeordnet sind, Licht von einem jeweiligen Strahler bei der jeweiligen Wellenlänge zu detektieren und ein Lichtsignal zu erzeugen, das das detektierte Licht darstellt, wobei die Empfänger durch einen Spalt (118) von den Strahlern beabstandet sind, durch den im Einsatz Öl strömen kann; und
einen Prozessor (124), der angeordnet ist, die jeweiligen Lichtsignale zu analysieren und vergleichen, um eine Menge an in dem Öl vorhandenen Wasser zu bestimmen, wobei der Prozessor (124) angeordnet ist, zu bestimmen, wenn eine Schwankung eines oder mehrerer der Lichtsignale einen voreingestellten Wert übersteigt.

2. System nach Anspruch 1, wobei der Spalt (118), der die Empfänger von den Strahlern trennt, kleiner als 2 mm, vorzugsweise kleiner als 1 mm und am meisten bevorzugt um 0,5 mm ist.

3. System nach Anspruch 1 oder Anspruch 2, wobei die Strahler (112A, 112B, 112C, 112D) Leuchtdioden sind und die Empfänger Fotodioden (122A, 122B, 122C, 122D) sind, die bei der entsprechenden Frequenz arbeiten.

4. System nach einem der vorhergehenden Ansprüche, wobei die Strahler in einen Eingang einer optischen Strahlerfaser (114) über einen optischen Strahlerschalter (115) gekoppelt sind, wobei die Empfänger mit einem Ausgang einer optischen Empfängerfaser (120) über einen optischen Empfängerschalter (119) gekoppelt sind, wobei der Spalt (118) zwischen einem Ausgang der optischen Strahlerfaser und einem Eingang der optischen Empfängerfaser bereitgestellt ist und der optische Strahlerschalter und der optische Empfängerschalter miteinander synchronisiert sind.

5. System nach einem der vorhergehenden Ansprüche, wobei der Prozessor angeordnet ist, die jeweiligen Lichtsignale zu vergleichen und die Menge an Wasser anhand der Lichtsignale einer Teilgruppe der Sensoren zu bestimmen.

6. System nach einem der vorhergehenden Ansprüche, wobei die erste Mehrzahl von Wellenlängen um 1250 nm und 1400 nm beträgt.

7. System nach einem der vorhergehenden Ansprüche, wobei die Anordnungen mindestens vier Strahler (112A, 112B, 112C, 112D) und mindestens vier Empfänger (122A, 122B, 122C, 122D) umfassen.

8. System nach einem der vorhergehenden Ansprüche, das in einer Ölzufuhrleitung zu einem mechanischen System angeordnet ist.

9. Verfahren zum Detektieren des Vorhandenseins von Wasser in Öl mithilfe des Systems nach einem der vorhergehenden Ansprüche, umfassend Strömen von Licht durch das Öl von den Strahlern (112A, 112B, 112C, 112D) zu den Empfängern (122A, 122B, 122C, 122D), Detektieren an den Empfängern des von den Strahlern übertragenen Lichts, um Lichtsignale zu erzeugen, die das detektierte Licht darstellen, Analysieren und Vergleichen der jeweiligen Lichtsignale, um eine Menge an in dem Öl vorhandenem Wasser zu bestimmen, Analysieren der Lichtsignale, um einen Schwankungsbetrag der Signale zu bestimmen, Überwachen des Schwankungsbetrags der Lichtsignale, um einen Lastsprung zu erkennen, der eine Sättigung des Öls darstellt, und Erzeugen eines Sättigungssignals, das eine Sättigung anzeigt, wenn mindestens eines der Lichtsignale eine Schwankung von mehr als einem vorbestimmten Wert anzeigt.

10. Verfahren nach Anspruch 9, wobei das Bestimmen eines Schwankungsbetrags Messen einer Spitze-zu-Spitze-Schwankung des Lichtsignals innerhalb eines Abtastzeitraums umfasst.

11. Verfahren nach Anspruch 10, wobei der Abtastzeitraum zwischen 0,5 Sekunden und 10 Sekunden, vorzugsweise zwischen 1 Sekunde und 3 Sekunden beträgt.

12. Verfahren nach Anspruch 10 oder Anspruch 11, wobei eine Sättigung erkannt wird, wenn ein Verhältnis der Lichtsignalschwankung in einem zweiten Abtastzeitraum zu der Lichtsignalschwankung in einem ersten Abtastzeitraum einen vorbestimmten Wert überschreitet.

13. Verfahren nach einem der Ansprüche 9 bis 12, ferner umfassend Bestimmen der Zeit, zu der das Öl über seinem Sättigungspegel bleibt.

## Revendications

1. Système pour détecter la présence d'eau dans l'huile, comprenant :
un réseau d'émetteurs infrarouges (112A, 112B, 112C, 122D) pour émettre de la lumière à une première pluralité de longueurs d'onde entre 1100 nm et 1500 nm ;
un réseau de récepteurs infrarouges (122A, 122B, 122C, 122D), chacun étant agencé pour détecter la lumière provenant d'un émetteur respectif à la longueur d'onde respective et pour produire un signal lumineux représentatif de la lumière détectée, les récepteurs étant espacés des émetteurs par un espace (118) à travers lequel l'huile utilisée peut passer ; et
un processeur (124) agencé pour analyser et comparer les signaux lumineux respectifs pour déterminer une quantité d'eau présente dans l'huile, le processeur (124) étant agencé pour déterminer quand une fluctuation d'un ou plusieurs des signaux lumineux dépasse une valeur préétablie.

2. Système selon la revendication 1, l'espace (118) séparant les récepteurs des émetteurs étant inférieur à 2 mm, de préférence inférieur à 1 mm et le plus préférablement autour de 0,5 mm.

3. Système selon la revendication 1 ou la revendication 2, les émetteurs (112A, 112B, 112C, 112D) étant des diodes électroluminescentes et les récepteurs étant des photodiodes (122A, 122B, 122C, 122D), fonctionnant à la fréquence correspondante.

4. Système selon l'une quelconque des revendications précédentes, les émetteurs étant couplés à une entrée d'une fibre optique d'émission (114) via un commutateur optique d'émission (115), les récepteurs étant couplés à une sortie d'une fibre optique de réception (120) via un commutateur optique de réception (119), l'espace (118) étant prévu entre une sortie de la fibre optique d'émission et une entrée de la fibre optique de réception, et le commutateur optique d'émission et le commutateur optique de réception étant synchronisés entre eux.

5. Système selon l'une quelconque des revendications précédentes, le processeur étant agencé pour comparer les signaux lumineux respectifs et déterminer la quantité d'eau sur la base des signaux lumineux d'un sous-groupe des capteurs.

6. Système selon l'une quelconque des revendications précédentes, la première pluralité de longueurs d'onde étant d'environ 1250 nm et 1400 nm.

7. Système selon l'une quelconque des revendications précédentes, les réseaux comprenant au moins quatre émetteurs (112A, 112B, 112C, 112D) et au moins quatre récepteurs (122A, 122B, 122C, 122D).

8. Système selon l'une quelconque des revendications précédentes, situé dans une conduite d'alimentation en huile d'un système mécanique.

9. Procédé pour détecter la présence d'eau dans l'huile en utilisant le système selon une revendication précédente, comprenant le passage de lumière à travers l'huile depuis les émetteurs (112A, 112B, 112C, 112D) vers les récepteurs (122A, 122B, 122C, 122D), la détection au niveau des récepteurs, de la lumière transmise depuis les émetteurs pour produire des signaux lumineux représentatifs de la lumière détectée, l'analyse et la comparaison des signaux lumineux respectifs pour déterminer une quantité d'eau présente dans l'huile, l'analyse des signaux lumineux pour déterminer une quantité de fluctuation des signaux, la surveillance de la quantité de fluctuation des signaux lumineux pour identifier un changement d'étape représentant la saturation de l'huile et la génération d'un signal de saturation indiquant la saturation lorsqu'au moins un des signaux lumineux indique une fluctuation supérieure à une valeur prédéfinie.

10. Procédé selon la revendication 9, la détermination d'une quantité de fluctuation comprenant la mesure d'une variation de crête à crête du signal lumineux à l'intérieur d'une période d'échantillonnage.

11. Procédé selon la revendication 10, la période d'échantillonnage étant entre 0,5 seconde et 10 secondes, de préférence entre 1 seconde et 3 secondes.

12. Procédé selon la revendication 10 ou la revendication 11, la saturation étant identifiée lorsqu'un rapport de la fluctuation du signal lumineux dans une deuxième période d'échantillonnage sur la fluctuation du signal lumineux dans une première période d'échantillonnage dépasse une valeur prédéterminée.

13. Procédé selon l'une quelconque des revendications 9 à 12, comprenant en outre la détermination du temps pendant lequel l'huile reste au-dessus de son niveau de saturation.
